(19) Europäisches Patentamt
European Patent Office
Office européen des brevets

(11) Publication number: **0 092 288**
**A2**

# (12) EUROPEAN PATENT APPLICATION

(21) Application number: **83200548.2**

(22) Date of filing: **18.04.83**

(51) Int. Cl.³: **C 07 D 471/04**
// (C07D471/04, 221/00, 209/00)

(30) Priority: **20.04.82 IT 2082282**

(43) Date of publication of application: **26.10.83**
**Bulletin 83/43**

(84) Designated Contracting States: **BE CH DE FR GB LI LU NL SE**

(71) Applicant: **ANIC S.p.A., Via Ruggero Settimo, 55, I-90139 Palermo (IT)**

(72) Inventor: **Chiusoli, Gian Paolo, Via Solferino 18bis, I-43100 Parma (IT)**
Inventor: **Pallini, Luciano, Via V. Bottego 3, I-43045 Fornovo Taro (Parma) (IT)**
Inventor: **Terenghi, Giuliana, Via C. Collodi 18, I-42024 Castelnovo Sotto (Reggio Emilia) (IT)**

(74) Representative: **Roggero, Sergio et al, Ing. Barzanò & Zanardo Milano S.p.A. Via Borgonuovo 10, I-20121 Milano (IT)**

(54) **Process for preparing 6-substituted 1,1,3,3-tetramethyl-1-H-2,3-dihydro-pyrrolo -(3,4-c)-pyridines, and products thus obtained.**

(57) A process for preparing 6-substituted 1,1,3,3-tetramethyl-1-H-2,3-dihydro-pyrrolo-[3,4-c]-pyridines, of general formulal

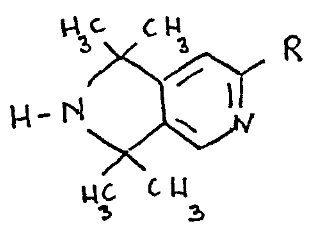

in which R can be an aryl containing up to 12 carbon atoms, or $CH_2R'$ where R' is an aryl of up to 12 carbon atoms or $CH_2R''$ where R' is an alkyl or aralkyl containing up to 20 carbon atoms, including branched, –OH, $–NH_2$ (including alkyl or acyl substituted), –CN or $COOR^{IV}$ where $R^{IV}$ is an alkyl containing up to 4 carbon atoms, consisting of reacting a tetramethyl-substituted dipropargylamine with nitriles, in an excess of the nitrile – which is possibly also used as the solvent – at a temperature of between 50 and 150 °C, in the presence of a catalyst prepared by the in-situ reduction of $CoCl_2$ with manganese or zinc dust, or by treating Raney cobalt with thiourea, and the products thus obtained.

EP 0 092 288 A2

The present invention relates to a process for preparing 6-substituted 1,1,3,3-tetramethyl-1-H-2,3-dihydro-pyrrolo-[3,4-c]-pyridines, of general formula:

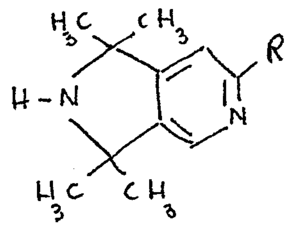

in which R can be an aryl containing up to 12 carbon atoms, $CH_2R'$ where R' is an aryl of up to 12 carbon atoms or $CH_2R"$ where R" is an alkyl or aralkyl containing up to 20 carbon atoms, including branched; -OH, $-NH_2$ (including alkyl or acyl substituted), -CN or $COOR^{IV}$, where $R^{IV}$ is alkyl containing up to 4 carbon atoms, consisting of reacting a tetramethyl-substituted dipropargylamine with a nitrile, in an excess of the nitrile, which is possibly also used as the solvent, at a temperature of between 50 and 150°C, in the presence of a catalyst prepared by the in-situ reduction of $CoCl_2$ with manganese or zinc dust, or by treating Raney cobalt with thiourea.

It is known to prepare pyrrolopyridines unsubstituted in positions 1 and 3 from unsubstituted diacetylene compounds in the presence of special monovalent cobalt complexes, but this catalytic system has proved ineffective for substituted dipropargylamines.

Moreover, unsubstituted dipropargylamine reacts with acetonitrile in the presence of cobalt carbonyl to give 6-methylated pyrrolopyridine only with a yield of 21.4%.

Furthermore, in the presence of acetonitrile (used as solvent) and acetylene compounds, $CoCl_2$/Mn gives rise to the trimerisation of these latter but without any interaction with the acetonitrile (Organometal

Chem. Syn. 1, 185-1971).

It has therefore been surprising to find that with the catalytic system proposed by the present invention, it is possible to obtain very positive results from substrates having a structure very similar to those used in the known art.

By carrying out the reaction under extremely moderate conditions, ie bringing the reagents into contact with the catalyst under agitation, preferably in an inert atmosphere, at a temperature of about 70-85°C, yields with respect to the fed dipropargylamine have been obtained which always exceed 80%.

The products obtained can be separated from the reaction mixture in a simple manner by conventional methods, preferably by distillation or crystallisation.

The products obtained are new chemical products which can be used as intermediates for organic synthesis, both for the production of pyridine derivatives and of pyrrole derivatives in all their fields of application.

By suitably varying the structure of the nitrile used, it is possible to obtain compounds which in position 6 contain the most suitable substituents for the use for which the product is intended.

The present invention will be more apparent from the examples given hereinafter for illustrative purposes only. Example 7 is particularly important, from which it appears that the reduced cobalt also has a catalytic effect in the heterogeneous phase.

EXAMPLE 1

32.4 mg (0.25 mmoles) of anhydrous $CoCl_2$, 7.5 ml of acetonitrile, 1.128 g (7.57 mmoles) of di-tert-propargylamine (DTPA) and 62.4 mg (1.134 mmoles) of finely divided Mn metal are fed successively into a 25 ml

flask under an inert atmosphere.

The reaction mixture is heated under vigorous agitation to 80°C by an oil bath for six hours. It is cooled to ambient temperature, hydrolysed and extracted with ethyl acetate. The organic extract is dried over anhydrous $Na_2SO_4$, filtered and analysed by g.l.c. and is found to contain almost exclusively 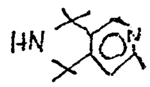 (MW 190).

The gas chromatography yield is 98% with respect to the fed DTPA, and the catalytic efficiency is 30 moles of product per mole of catalyst. The product, which can be easily crystallised from diethyl ether, has a melting point of 92-93°C.

Maximum catalytic efficiency (50) was obtained with a reduction in the yield (25%), but not in the selectivity (98%).

Good results are obtained by carrying out the reaction in anisole, chlorobenzene, ethyl acetate or tetrahydrofuran.

EXAMPLE 2

23 mg (0.177 mmoles) of anhydrous $CoCl_2$, 5.4 ml of acetonitrile, 0.813 g (5.45 mmoles) of DTPA and 50 mg (0.77 mmoles) of Zn dust are reacted under the same conditions as Example 1.

After about ten hours at 80°C, the reaction mixture is hydrolysed, extracted, dried and analysed as in Example 1.

The gas chromatography yield of the product 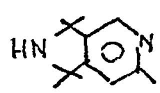 is 97% with respect to the fed DTPA, with a catalytic efficiency of 30 moles of product per mole of catalyst.

Like results can be obtained by using $CoCl_2.6H_2O$ in place of anhydrous $CoCl_2$.

EXAMPLE 3

25 mg (0.19 mmoles) of anhydrous $CoCl_2$, 6 ml of n-butyronitrile, 1.26 g (8.46 mmoles) of DTPA and 44 mg (0.8 mmoles) of Mn metal are

reacted under the same conditions as Example 1.

After 20 hours at 75°C, the reaction mixture is treated as in Example 1.

The gas chromatography yield of the product 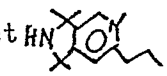 (MW 218) is 91% with respect to the fed DTPA, and the catalytic efficiency is 40 moles of product per mole of catalyst.

The product, which can be crystallised from diethyl ether, has a melting point of 52-53°C.

EXAMPLE 4

16.5 mg (0.127 mmoles) of anhydrous $CoCl_2$, 6 ml of benzonitrile, 0.95 g (6.38 mmoles) of DTPA and 35 mg (0.63 mmoles) of Mn metal are reacted under the same conditions as Example 1.

After 20 hours at 75°C, the reaction mixture is treated as in Example 1.

The gas chromatography yield of the product 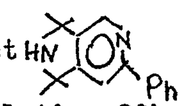 (MW 252) is 26% with respect to the fed DTPA, and the catalytic efficiency is 13 moles of product per mole of catalyst.

The product, distilled with a bulb furnace (135-140°C / 1-1.2 mmHg) has a melting point of 55-57°C.

EXAMPLE 5

28 mg (0.215 mmoles) of anhydrous $CoCl_2$, 3 ml of phenylacetonitrile, 0.96 g (6.44 mmoles) of DTPA and 36 mg (0.65 mmoles) of Mn metal are reacted under the same conditions as Example 1.

After 20 hours at 75°C, the reaction mixture is treated as in Example 1.

The gas chromatography yield of the product 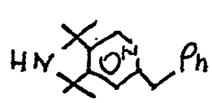 (MW 268) is 93% with respect to the fed DTPA, and the catalytic efficiency is 28 moles of product per mole of catalyst.

The product, distilled with a bulb furnace (135-140°C / 0.5-0.7 mmHg), has a melting point of 61-63°C.

EXAMPLE 6

40 mg (0.307 mmoles) of anhydrous $CoCl_2$, 4 ml of anisole, 1.875 g (23.42 mmoles) of succinodinitrile, 1.15 g (7.7 mmoles) of DTPA and 70 mg (1.27 mmoles) of Mn metal are fed successively under an inert atmosphere into a 25 ml flask.

The reaction mixture is heated under agitation to 80°C by means of an oil bath for 48 hours. It is cooled, hydrolysed, extracted with ethyl acetate, and the organic extracts are dried over anhydrous $Na_2SO_4$, and subjected to gas chromatography analysis and determination.

The gas chromatography yield of the product 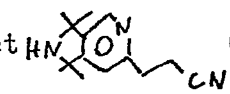 (MW 229) is 80% with respect to the fed DTPA, and the catalytic efficiency is 20 moles of product per mole of catalyst.

The product can be easily crystallised from diethyl ether after acid-basic extraction of the reaction mixture. Melting point 91-93°C.

Like results can be obtained by reacting adipodinitrile instead of succinodinitrile. The product obtained in this case is 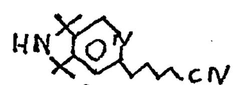 (MW 257), which on distillation with a bulb furnace (145-155°C / 0.9-1 mmHg) has a melting point of 40-41°C.

EXAMPLE 7

0.5 ml of Raney cobalt suspended in ethanol, and 100 mg of thiourea are fed into a 25 ml flask. When gas ceases to be evolved, the alcohol is eliminated by washing three times with acetonitrile, and leaving 4 ml of it at the end.

0.820 g (5.5 mmoles) of DTPA are then added.

The reaction mixture is heated under vigorous agitation to 80°C by means of an oil bath for three days. It is then allowed to cool to

ambient temperature, filtered, the precipitate washed repeatedly, hydrolysed, extracted with ethyl acetate, and then subjected to gas chromatography analysis.

The yield of the product 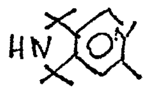 is 17% with respect to the fed DTPA, most of which remains unaltered.

CLAIMS

1. 6-substituted 1,1,3,3-tetramethyl-1-H-2,3-dihydro-pyrrolo-[3,4-c]-pyridines of general formula:

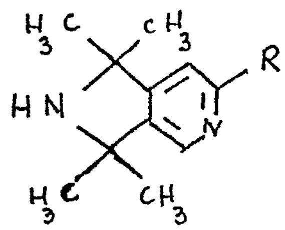

in which R can be an aryl containing up to 12 carbon atoms, $CH_2R'$ where R' is an aryl of up to 12 carbon atoms or $CH_2R''$ where R" is an alkyl or aralkyl containing up to 20 carbon atoms, including branched, -OH, $-NH_2$ (including alkyl or acyl substituted), -CN or $COOR^{IV}$, where $R^{IV}$ is an alkyl containing up to 4 carbon atoms.

2. A process for preparing the compounds as claimed in claim 1, consisting of reacting a tetramethyl-substituted dipropargylamine with a nitrile in the presence of a catalyst constituted by the product of the reduction of $CoCl_2$ with manganese or zinc dust.

3. A process for preparing compounds as claimed in claim 1, consisting of reacting a tetramethyl-substituted dipropargylamine with a nitrile in the presence of a catalyst constituted by Raney cobalt treated with thiourea.

4. A process as claimed in the preceding claims, characterised in that the reaction is carried out at a temperature of between 50 and 150°C.

5. A process as claimed in claims 2 and 3, characterised in that the reaction is carried out in the presence of an excess of nitrile.